# EUROPEAN PATENT APPLICATION

(11) **EP 4 053 298 A1**
(43) Date of publication of application: **07.09.2022**
(21) Application number: 20881378.2
(22) Date of filing: 28.10.2020
(51) Int. Cl.: C22B 3/18, C12N 1/20

(54) **METHOD FOR BIODISINTEGRATING SCRAP METAL USING A BACTERIAL CONSORTIUM ADAPTED TO HIGH CONCENTRATIONS OF IRON(II) SULFATE AND IRON(III) SULFATE**

(30) Priority: 28.10.2019 CL 20193094
(71) Applicant: Rudanac Biotec SpA, Antofagasta (CL)
(72) Inventor: REALES DEL CANTO, Nadac Selec Keren, Antofagasta (CL)
(74) Representative: Pons
(86) International application number: PCT/CL2020/050143
(87) International publication number: WO 2021/081682

(57) **Abstract**

Method of biodesintegrating metal scrap with a bacterial consortium adapted to high concentrations of ferrous sulfate and ferric sulfate, access RGM 2972 of the Chilean Collection of Genetic and Microbial Resources; intermediate solution comprising it, useful in eliminating surface oxidation in metallic structure; and oxidizing solution, useful in the hydrometallurgical extraction of copper.

SEQUENCE LISTING

<110>  Rudanac Biotec SpA

<120>  METHOD OF BIODESINTEGRATING METAL SCRAP WITH A
BACTERIAL CONSORTIUM ADAPTED TO HIGH CONCENTRATIONS OF FERROUS
SULPHATE AND FERRIC SULPHATE

<130>  PE775PCTORIB

<160>  4

<170>  PatentIn version 3.5

<210>  1
<211>  20
<212>  DNA
<213>  Artificial Sequence

<220>
<223>  Primer, oligonucleotide sequence

<400>  1
agagtttgat cctggctcag                                                   20


<210>  2
<211>  20
<212>  DNA
<213>  Artificial Sequence

<220>
<223>  Primer, oligonucleotide sequence

<400>  2
agaaaggagg tgatccagcc                                                   20


<210>  3
<211>  20
<212>  DNA
<213>  Artificial Sequence

<220>
<223>  Primer, oligonucleotide sequence

<400>  3
ggaatcttcc acaatgggcg                                                   20


<210>  4
<211>  20
<212>  DNA
<213>  Artificial Sequence

<220>
<223>  Primer, oligonucleotide sequence

<400>  4
ttccccacgc gttactcacc                                                   20

## Description

### FIELD OF THE INVENTION

The invention relates to the disposal of industrial waste and the circular economy of scrap metal. In particular, a method that allows the bio-disintegration of industrial metal scrap, using microorganisms that feed on the metal, without generating polluting waste, being an environmentally friendly process, it is economically profitable, easy to implement and scale. This process generates two by-products: 1) an intermediate solution of the solution that Biodesintegrates the metal scrap, which allows to eliminate the surface oxide of a metallic structure and 2) a highly oxidizing final solution with application in the hydrometallurgy process in the extraction of copper.

### ANTECEDENTS

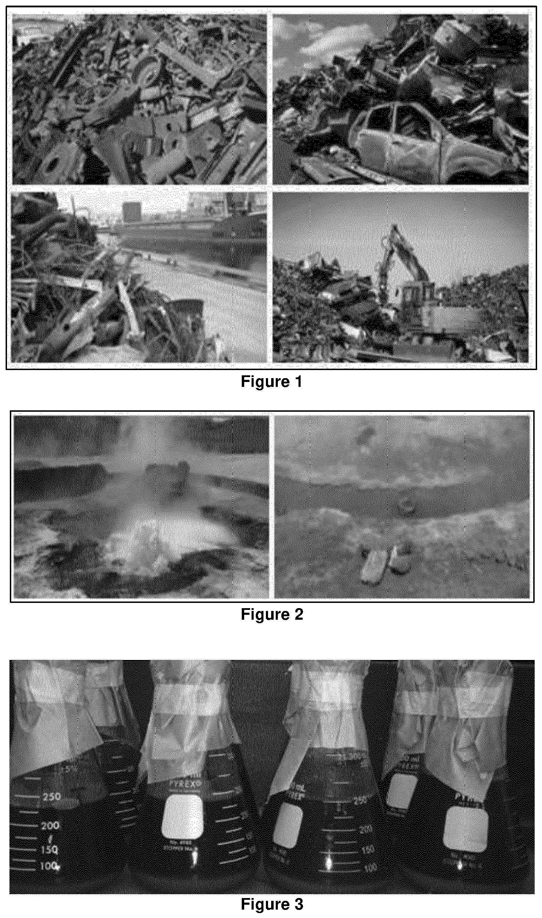

The generation of industrial waste is one of the serious environmental problems that the planet is experiencing, driven by rapid urbanization and population growth. The annual generation of waste worldwide is expected to increase by 70%, valued at 3,400 million tons in the next 30 years, compared to 2,010 million tons in 2016. Among industrial waste, is metal scrap (Figure 1), generated by the cemeteries of vehicles, the mining industry, industrial machinery, construction structures, ships, containers and the railway industry and others. These wastes are usually stored in landfills, generating a great impact on the environment, human health, flora and fauna, due to the dispersion in the ecosystem of metallic particles (Fe, Mn, Zn, Cu and Pb) when exposed to different climatic conditions, according to data provided by the World Health Organization (WHO) and the United States Environmental Protection Agency (USEPA).

Heavy metals are economically important in industrial use and the most important pollutants in the environment. Environmental contamination by heavy metals has become a serious threat to living organisms in the ecosystem (Hrynkiewicz K., Baum C. Application of microorganisms in bioremediation of environment from heavy metals. Environmental Deterioration and Human Health: Natural and Anthropogenic Determinants. 2014: 215-227). The toxicity of the metal is of great environmental concern due to its bioaccumulation and its non-biodegradability in nature (Wai WL, Kyaw NAK, Nway NHN Biosorption of Lead (Pb2 +) by using Chlorella vulgaris. Proceedings of the International Conference on Chemical engineering and its applications (ICCEA); 2012; Bangkok (Thailand). Various inorganic metals such as magnesium (Mg), nickel (Ni), chromium (Cr3 +), copper (Cu), calcium (Ca), manganese (Mn) and sodium (Na ), as well as zinc (Zn) are vital elements necessary in small amounts for metabolic and redox functions. Heavy metals such as aluminum (Al), lead (Pb), cadmium (Cd), gold (Au), Mercury (Hg) and silver (Ag) have no biological role and are toxic to living organisms (Turpeinen R., Kairesalo T., Haggblom M. Microbial activity community structure in arsenic, chromium and copper contaminated soils. Journal of Environmental Microbiology. 2002; 35 (6): 998-1002. Doi: 10.1016 / S0168-6496 (03) 0 0232-0).

The contamination of surface waters with heavy metals from industrial activities, especially those from junkyards, has caused a great threat to human life, exposing man to a series of dangers, diseases, disabilities and, consequently, death.. This study focuses on the water quality indices of the Owode-Onirin and Lafenwa junkyard with respect to its physicochemical parameters and heavy metal concentrations by evaluating the heavy metal contamination index (HPI), the metal index ( MI) and the potential ecological risk index (PERI). Weiss (1974), observes that groundwater contamination necessarily deteriorates water quality, resulting in public health risks, Ogbonna et al. (2006) also emphasized that it will negatively affect said water for domestic, agricultural, industrial and municipal use (Akhilesh et al. 2009).

Heavy metal research is very essential according to Yahaya et al. (2009), since small modifications in its concentration above threshold levels for biogenic or anthropogenic factors lead to serious environmental danger and subsequent health problems. Klavins et al. (2000), Tam and Wong (2000), Yuan et al. (2004), Hakan (2006) reiterated that heavy metals are serious environmental pollutants with a tendency to toxicity, longevity and persistence in the environment. Environmental pollution by heavy metal ions arises as a result of many activities in the environment. In the soil system, toxic metal contamination is due to both biogenic processes (mineral weathering) and anthropogenic activities (agriculture, burning of fossil fuels, industry, junkyards, vehicular emission, mining and metallurgical processes and disposal of debris) as investigated by Kumar (2005), Biasioli et al. (2006) and Martin at al. (1982) concluding that heavy metal contamination in the soil-water-plant ecosystem is of great importance due to the possible influence on the food chain (Gray et al. 2003).

Heavy metal pollutants could be chemical and biological processes in nature with a potential impact on human health and environmental well-being (Giuliano et al. 2007). The presence of heavy metals in and around urban areas has been an area of great concern due to its long and persistent nature and its long biological life within the human system when taken. The negative effects due to heavy metal contamination in surface and groundwater are well established by Tumuklu et al. (2007) for manganese, chromium and zinc that cause neurosis and chlorosis, while nickel, cobalt and cadmium hinder stomatal activity and decrease photosynthesis in plants (Prasad 1995). Aluminum, cobalt, copper, iron, lead, manganese, nickel, and zinc have been reported to cause potential hazards in water (Grigalaviciene et al. 2005; Tumuklu et al. 2007; Al-Kashman and Shawabkeh 2009).

Akoto et al. (2008) in their finding documented that in most developing countries, junkyards are increasing and will continue to expand from rapid economic growth through increasing population, industrialization, and increased motorization. Although the distribution and concentration of heavy metals in soluble water is well documented for several places in developed countries, there is a paucity of information from less developed countries.

Scrap recycling would make a significant contribution to not worsening the current environmental environment. However, scrap metal processing facilities have been identified as a source of emissions of mercury (Hg) and other heavy metals, due to the smelting process. In addition, there are certain types of scrap that by their nature cannot be recycled due to the presence of gases, oils, rubber, sealed parts, shock absorbers, radioactive material, toxic, excess earth, pieces of concrete or bricks, adhered, chemical elements (Cu, Cr, Ni, Sn, P, I, Pb), asbestos, rubber, asphalt and other polymers. All of them, in excess amounts, cause problems for steel, such as lowering its ductility and weldability. Likewise, its presence in scrap metal makes it difficult to handle when melted, remaining permanently present in the steel, rendering its application useless.

Due to the antecedents presented above, air, water and soil contamination by heavy metals will increase because there is no solution that allows the total elimination of the pollutants generated by scrap metal. Scrap metal recycling is a partial solution that reuses metal waste but does not eliminate environmental pollution, since in its foundry processes, it expels large amounts of heavy metals into the environment. In addition, it must be considered that not all metallic waste can be recycled, many of which are stored in landfills, which ultimately contributes to maintaining high levels of environmental pollution. Technologies have currently been developed that allow the removal of heavy metal contamination from the soil, using microorganisms in the processes, as described by Kapahi and Sachdeva (2017). However, this solution is applicable once the heavy metal is already on the soil surface, a process called Bioremediation (Mycoremediation potential of Pleurotus species for heavy metals: a review. 2017)

Although no documents were identified that describe a method and/or product used for the biodesintegration of scrap metal, some patent revisions that use methods and/or devices to recycle and reuse scrap metal are described below.

Document WO2015099529 describes a method for processing asbestos-containing scrap steel into useful products, products that can be handled safely. In accordance with the present invention, asbestos-containing steel scrap is melted in a furnace, which results in the destruction of the asbestos fibers. It has been found possible to carry out such a process in an economically viable manner. According to the invention, the asbestos-containing steel is heated to a high temperature so that the steel melts. As a result, asbestos will become harmless material, allowing for safe handling and processing of the resulting products.

Document WO2016125115 describes a plant and a method for recovering metals and / or metal oxides from industrial process residues, in particular residues of petroleum refining products (refinery residues).

Document WO2018137957 refers to a method for detinning steel scrap, comprising the following steps: providing organically contaminated tinned steel scrap, cleaning the organically contaminated tinned steel scrap so that a clean tinned steel scrap is obtained, in which The scrap is exposed to an inert atmosphere containing sulfur, so that a tin-sulfur phase forms on the surface of the steel scrap, separating the tin-sulfur phase from the steel scrap, so that a fraction tin-sulfur and a fraction of purchased debonded steel scrap.

Document DE29821781 refers to a device for the production of non-ferrous metal from articles consisting of or containing ferrous and non-ferrous metal, the device comprises a furnace to heat the article to a separation temperature located between the melting temperatures of the ferrous and non-ferrous metal and a collecting device to collect the molten non-ferrous metal.

Document CN109870019 describes a production line for high temperature drying treatment of steel scrap containing oil. The high temperature drying treatment production line comprises a crusher, a feeding system, a heating furnace, a discharge system, a smoke treatment system and a control system; the feeding system and the discharge system are connected with the control system, and a waste gas pipe is arranged between the heating furnace and the smoke treatment system; the smoke treatment system comprises a settling dust remover, a cooling tower and an electrostatic device; and the settling dust remover and heating furnace are connected through the waste gas pipeline. The high-temperature drying treatment production line adopts a complete set of technology and device that takes a high-temperature drying and washing technology as its core, integrating the automatic feeding and discharge systems and the high-temperature smoke treatment system. temperature and is used for efficient and clean cleaning and automated treatment of large and medium batches of oil-containing steel scrap, automatic batch drying is achieved, drying efficiency is high, environmental pollution is effectively avoided, Potential safety hazard is eliminated, promoted car remanufacturing industry development is developed, and social damage caused by automobile waste is eliminated to some extent.

Document CN109870019 describes a production line for high temperature drying treatment of steel scrap containing oil. The high temperature drying treatment production line comprises a crusher, a feeding system, a heating furnace, a discharge system, a smoke treatment system and a control system; the feeding system and the discharge system are connected with the control system, and a waste gas pipe is arranged between the heating furnace and the smoke treatment system; the smoke treatment system comprises a settling dust remover, a cooling tower and an electrostatic device; and the settling dust remover and heating furnace are connected through the waste gas pipeline. The high-temperature drying treatment production line adopts a complete set of technology and device that takes a high-temperature drying and washing technology as its core, integrating the automatic feeding and discharge systems and the high-temperature smoke treatment system. temperature and is used for efficient and clean cleaning and automated treatment of large and medium batches of oil-containing steel scrap, automatic batch drying is achieved, drying efficiency is high, environmental pollution is effectively avoided, Potential safety hazard is eliminated, promoted car remanufacturing industry development is developed, and social damage caused by automobile waste is eliminated to some extent.

Document CN109746225 describes a scrap cleaning machine comprising a housing, a blow port, a scrap collector box, a first pressure regulating valve, a second pressure regulating valve, a pneumatic valve, a blow gun interface powder, a powder blower gun, an air motor, rotating blades and an intake tube. The casing is square in shape, the upper part of the casing is provided with the blowing port, the inside of the casing is provided with the air motor, rotating blades and the scrap collection box, the air motor is located under the port of blowing, the air motor and rotating blades are connected, the scrap collector is installed at the bottom of the casing, and the scrap collector is inserted into the casing from the outer surface of one side of the casing in the form drawer; and the first pressure regulating valve, the second pressure regulating valve and the intake pipe are embedded in the outer surface of the other side of the shell, the intake pipe is installed between the two pressure regulating valves, the pneumatic valve is installed in the intake pipe, and the intake pipe is connected with the left and right pressure regulating valves to guide the external gas to the pressure regulating valves.

The present invention makes it possible to overcome the difficulties mentioned at the beginning, by describing a process that biodesintegrates metal scrap using a biological solution, which does not generate polluting waste, is a friendly process with the environment, is economically profitable, easy to implement and easy to use. climb. This solution allows the disintegration of metal scrap by more than 60%, allowing the total elimination of industrial waste, using a consortium **RGM 2972** of October 16, 2019 (Chilean Collection of Genetic and Microbial Resources) well adapted to conditions of high tolerance to ferrous sulfate (FeSO₄) and ferric sulfate (Fe₂(SO₄)₃). Furthermore, the present invention is characterized by having two more solutions: 1) an intermediate solution, which allows to eliminate the surface oxide of a metallic structure and 2) a highly oxidizing final solution with application in the hydrometallurgy process in the extraction of copper. On the other hand, the other patents use different physical and / or chemical procedures that reuse metal scrap, without eliminating the contamination of heavy metals to the environment.

### BRIEF DESCRIPTION OF THE INVENTION

The present invention solves the aforementioned problem by means of a consortium of autotrophic microbials with the ability to tolerate high concentrations of ferrous sulfate and ferric sulfate to bio-disintegrate scrap metal in an acid solution. The microbial consortium was isolated and cultured in chemolytoautotrophic medium (9K) and then analyzed by confocal and electron microscopy (STEM). Phylogenetic diversity was determined by PCR-DGGE and sequencing, to finally evaluate its tolerance to ferrous sulfate (FeSO₄) and ferric sulfate (Fe₂(SO₄)₃) and the effectiveness in the bio-disintegration process of metal scrap in more than 60 %, allowing the total elimination of industrial waste. The adaptation of the microbial consortium **RGM 2972** of October 16, 2019 (Chilean Collection of Genetic and Microbial Resources, to the different concentrations of ferrous sulfate (FeSO₄) was gradual (30, 40, 50, 60, 70, 80, 90 until exceeding 180% w/v) at a pH of 1.0 to 3.5, at a constant agitation that varies between 50 to 200 rpm, maintaining a temperature of 25ºC ± 5ºC, until each adaptation reaches a bacterial growth about 10⁸ cells/ml.

The results indicated that the bacterial consortium is made up of phyllotype related to species of the genus Leotospirillum, being able to grow in all ranges of ferrous sulfate (FeSO₄) and ferric sulfate (Fe₂(SO₄)₃) evaluated, tolerating concentrations of (FeSO₄) and (Fe₂(SO₄)₃) over 180% w/v in the 9K culture medium, reaching a population of over 10⁸ cells/ml in each adaptation. Two experimental tests were carried out to determine the degree of biodisintegration of the metal scrap in the presence of the adapted bacterial consortium.
- The first test consisted of determining the bio-disintegration of the metal scrap in the 9K culture medium, adding the inoculum of adapted bacteria. The consortium was able to biodisintegrate the metal scrap into 5%, 15%, 19%, 21%, 25%, 29% of the total weight of the metal scrap, in 7 days. The consortium was able to eliminate the surface oxidation of the metal scrap, due to the bioavailability of its energy source (FeSO₄), which they had to obtain directly from the metal scrap, increasing the metal's disintegration time.
- The second test consisted of determining the bio-disintegration of the metal scrap in the 9K culture medium enriched with high concentrations of ferrous sulfate (FeSO₄), adding the inoculum of adapted bacteria, then all the (FeSO₄) was transformed into (Fe₂(SO₄)₃). The consortium was able to biodisintegrate the metal scrap into 15%, 30%, 40%, 50%, 58%, 62% of the total weight of the metal scrap, in 7 days. The consortium was able to biodisintegrate its weight by 50% in just 5 days of experimentation. This was possible because the structure of the metal scrap was directly attacked by the action of the adapted bacteria and indirectly by the action (Fe₂(SO₄)₃). The biodesintegration of metal scrap in the presence of the bacteria characterized in the present invention generates a highly oxidizing final product, which can be used in the hydrometallurgy process, in the extraction of copper, commonly called the bioleaching process of copper or biological leaching. copper.

Thus, the present invention refers to a method to Biodisintegrate metal scrap in a 9K culture medium enriched with ferrous sulfate (FeSO₄) and using a consortium of bacteria tolerant to ferrous sulfate (FeSO₄) and ferric sulfate (Fe₂(SO₄)₃) - access number of the Bank of Genetic and Microbial Resources, Chile, **RGM 2972** of October 16, 2019 (Chilean Collection of Genetic and Microbial Resources, which includes species of the genus Leotospirillum.

The consortium - access number of the Microbial Genetic Resources Bank, Chile, **RGM 2972** of October 16, 2019 (Chilean Collection of Genetic and Microbial Resources, which includes species of the genus Leotospirillum, has the ability to grow in a 9K culture medium enriched with a high concentration of ferrous sulfate (FeSO₄) for its cellular development; oxidizing Fe⁺² to Fe⁺³ and in turn tolerating the high concentrations of ferric sulfate (Fe₂(SO₄)₃) generated, producing a solution that biodesintegrates the scrap metal and more than 60% of the total weight of the metal in 7 days.

### BREIF DESCRIPTION OF DRAWINGS

**Figure 1****.** Scrap metal, the cause of environmental pollution.
**Figure 2****.** Photographs of the sampling points in the EI Tatio-Chile geothermal field. Image on the left side, characterizes the sample taken from the edges of ponds or water pools and the image on the right side, characterizes the sample taken from the outlet channels.
**Figure 3****.** Image representing the growth of the initial bacterial consortium that was grown in medium 9K.
**Figure 4****.** Microscopic visualization of the cells that were treated with the Gram stain, to identify the type of bacteria present in the sample (Gram positive or Gram negative bacteria).
**Figure 5****.** A confocal electron microscope (STEM) was used to observe the bacterial inoculum adapted to high concentrations of ferrous sulfate (FeSO₄) and ferric sulfate (Fe₂(SO₄)3), highlighting the presence of mobile bacilli in liquid medium.

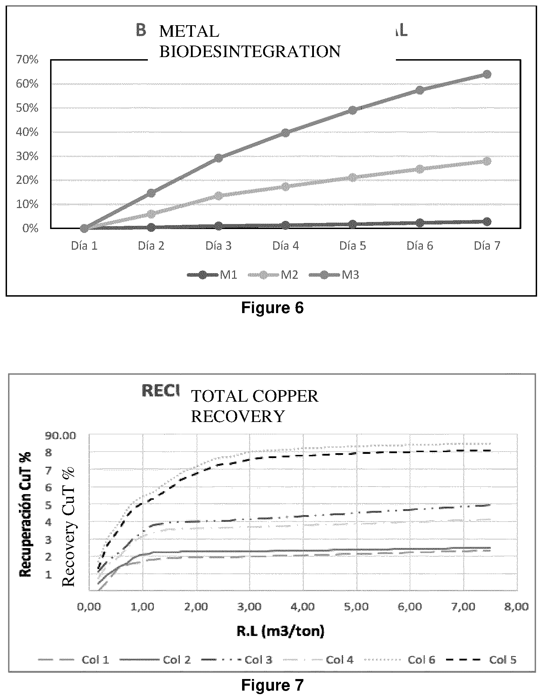

**Figure 6****.** Results of metal biodesintegration by bacterial effect adapted to high concentrations of ferrous sulfate (FeSO₄) and ferric sulfate (Fe₂(SO₄)₃). M1: Represents the negative control and the effect obtained with the 9K culture medium in an acidic environment is visualized, M2: Represents the experimental analysis 2 where the 9K culture medium in acid medium was used, which contained the adapted bacterial inoculum at high concentrations of ferrous sulfate (FeSO₄) and ferric sulfate (Fe₂(SO₄)₃), where the metal scrap was completely submerged in this solution and M3: Represents experimental analysis 3 where the 9K culture medium enriched with Ferrous sulfate in acid medium, the bacterial bacterial inoculum adapted to high concentrations of ferrous sulfate (FeSO₄) and ferric sulfate (Fe₂(SO₄)₃) was added and the culture medium was oxidized, later the metal scrap was submerged totally in this solution.
**Figure 7****.** Describes the total copper recovery obtained in 6 experimental tests carried out on 1.5 meter high columns, where the tests are: Column 1 and 2 represent the negative controls, columns 3 and 4 represent the experimental tests where it is added Bacterial inoculum (inoculum 1), in the mineral agglomeration process, whose bacteria are traditionally used in the biological leaching process and columns 5 and 6 represent the experimental tests where the solution obtained after the biodisintegration process of the Metal scrap, which contains bacteria adapted to high concentrations of ferrous sulfate (FeSO₄) and ferric sulfate (Fe₂(SO₄)₃), also whose solution has a high concentration of ferric sulfate (Fe₂(SO₄)₃) (Inoculum 2).

### Detailed Description of the Invention

There is growing public concern about the possible accumulation of heavy metals in the soil, due to rapid industrial development. In an effort to describe the state of soil contamination from industrial activities, Kabir et al. (2012) examined and reviewed relevant data reported by many studies. The results indicate that the soils were more significantly contaminated by metals such as lead, zinc, copper and cadmium. If the dominant species are evaluated by the highest mean concentration observed for different types of industry, the results are grouped into Pb, Zn, Ni, Cu, Fe and As in the foundry and metal production industries, Mn and Cd in the textile industry, and Cr in the leather industry. In most cases, metal levels in the studied areas were found to exceed the levels of common regulatory guidelines applied by many countries. The geoaccumulation index, calculated to estimate the enrichment of the concentrations of metals in the soil, showed that the level of contamination by metals in most of the areas studied is significant, especially for Pb and Cd. Therefore, it is important to maintain systematic and continuous monitoring of heavy metals and their derivatives to manage and suppress such contamination.

Industrial waste includes metal scrap, generated by vehicle cemeteries, the mining industry, industrial machinery, construction structures, ships, containers and the railway industry, and others. Metal scrap from out-of-circulation vehicles does not contain reusable materials and no type of treatment is given to this waste generated by units that are no longer in use. Unfortunately, all cities have specific places where huge amounts of junk are dumped. In this way, junkyards become a very important point of contamination. In addition, there are not only stopped vehicles but also dismantled cars that do not receive any type of treatment, thus generating large amounts of polluting garbage. The main cause of contamination is the exposure that this scrap has and the number of years it has been there. These car wrecks rust and damage the earth. The same happens if this rusty scrap comes into contact with water. When this scrap begins its oxidation process, lead and mercury particles are formed and take hundreds of years to decompose.

Unfortunately, the amounts of scrap that are produced in the world are extremely high and there is still no treatment for this problem. Only 43% of scrap is recycled out of the nearly one billion tons produced each year. In the recycling process, the contaminated metal scrap must go through a previous treatment in order that the Recycle the scrap metal collaborates in preventing the contamination from worsening and the poisoning of the water, soil and air is prevented by contact with any of the rusty metals.

Another cause of contamination due to the recovery of metal scrap is the Pickling baths, the pickling process aims to eliminate the metal oxides, the manufacturing scale, the annealing oxide and the rust from the surface of the metal part. the pieces perfectly clean. Most pickling tanks initially contain diluted 14-16% hydrochloric acid by weight. But as the bath is used, the concentration of hydrochloric acid decreases, a fact that requires periodic additions of acid so that the pickling speed does not drop significantly. The system remains like this until the solubility limit of ferrous chloride (FeCl₂) in the hydrochloric acid itself is reached, at which point the bath is exhausted and it is not possible to continue stripping. The exhausted bath contains an iron concentration equal to or greater than 140-150 g/L and must be renewed by a fresh bath. In those cases in which the industry carries out hot galvanizing processes, the exhausted bath in addition to iron also contains high levels of zinc (around 25 g/L).

The contamination produced by pickling baths when cleaning metal sheets with acids and bases, considered toxic, harmful and dangerous for health, infrastructure and the environment has increased considerably in recent years. The heavy metals such as zinc, chromium, and copper that accumulate in the pickling baths are theoretically considered as suspended substances and are considered as another problem due to the serious environmental pollution they produce (C. Frias and O. Perez, "Recuperación de acids and metals in exhausted baths from steel pickling "Revista de Metallurgia, vol. 34, pp. 427-431, 1998). In cleaning sheet metal surfaces, ISO 68 oil exhibits the highest average hydrocarbon contamination with 2,200.4 ppm at an average contact angle of 16.53 °. ISO 220 oil has higher average contamination of 0.51% carbon monoxide and 0.38% carbon dioxide.

Due to all the environmental pollution problems generated in the bad recycling processes of metal scrap, the exposure of heavy metals in the smelting process, the accumulation of metal scrap for years, the pickling baths in the cleaning processes of metal sheets, for reuse and other processes, are responsible for the serious problem of environmental pollution that our planet suffers and that currently there is no technology that can biologically eliminate this problem. Due to this, the present invention refers to a biotechnological solution capable of biodesintegrating metal scrap to avoid this type of contamination.

A consortium of bacteria capable of disintegrating metal scrap intervenes in the biodesintegration process. These microorganisms are characterized by being autotrophic, aerobic and chemosynthetic. This last characteristic makes them capable of biooxidation of metallic iron. (Souza et al., 2007). The autotrophic capacity allows them to synthesize their cellular components from inorganic compounds, such as the fixation of carbon dioxide (CO₂) from the atmosphere. They feed on the minerals from which they obtain energy and perform this task as part of their metabolic processes. They are also characterized by being organisms that live in extreme conditions (extremophiles): acidic pH and high concentrations of metals (Salo-Zieman et al., 2006). All these characteristics confer them the classification of iron oxidizing chemilithoautotrophic bacteria (Salo-Zieman et al., 2006).

Each species of bacteria has different nutritional requirements as energy sources (Table 1), so that a consortium of bacteria could be more beneficial in the biodisintegration process.

**Table 1. Bacteria with greater development under biooxidation conditions**

| Organisms | Temperature, °C | Carbon Source | Substrate |
|---|---|---|---|
| *Acidithiobacillus ferroxidans* | 20-50 | CO₂ | Fe²⁺, S° |
| *Acidithiobacillus thiooxidans* | 20-35 | CO₂ | S° |
| *Leptospirillum ferrooxidans* | 20-35 | CO₂ | Fe²⁺ |
| Género sulfolobus | 60-80 | CO₂ | Fe²⁺, S° |

The role that environmental, biological and physicochemical factors play on the growth and development of bacteria is fundamental in the performance of biodeintegration. The control of these factors is very important to ensure the optimal conditions of pH, humidity, temperature, nutrients, and energy sources that must exist together with the absence of inhibitors, which allow to obtain the maximum performance of the bio-disintegration.

The factors that influence the response of the microorganisms responsible for biodegradation are:
- **pH:** The pH in values 1.5 to 2.5 is optimal for the development of acidophilic bacteria, variations by enzymes or below the pH range of 1.5 to 2.5 cause bacterial growth to be inhibited.
- **Oxygen and carbon dioxide:** Oxygen is important to allow the oxidation of reduced substrates and in this way the microorganisms obtain the energy to survive. Air provides oxygen (O₂) and carbon dioxide (CO₂), the latter is important because it constitutes a source of carbon necessary for oxidation.
- **Nutrients:** Like all living beings, these microorganisms require nutritional sources for their optimal development, coming from the same metal scrap.
- **Energy Source:** microorganisms use the ferrous ion as their primary source of energy. In the biodesintegration of metal scrap, ferrous ion (Fe⁺²) is biologically extracted, therefore it is not necessary to add it.
- **Temperature:** Microorganisms are classified according to the temperature range in which they can survive. Thus mesophilic bacteria survive in an optimal temperature range between 30-40 ° C, moderately thermophilic bacteria survive at a temperature close to 50 ° C, and extremely thermophilic bacteria survive at a temperature above 65 ° C. If the temperature of the medium in which the microorganisms are found is less than 5 ° C, they become inactive, returning to fulfill their function if the temperature increases, but if the temperature of the medium exceeds the optimum, the microorganisms die (Nagpal et al., 2007 and Murr et al., 2008).
- **Redox Potential (Eh):** The oxidation of the reduced species depends on the transfer of electrons, therefore it influences the metabolism of the bacteria. In this way, the potential measurement is an indicator of microbial activity, the higher the measured potential, the higher the microbial activity. The optimal potential is 500 to 800 mV.

In the biodesintegration process using the bacteria described in the present invention, it is used to eliminate metal scrap, whose waste is generated because it has reached its useful life, the metal structure has undergone a destructive and functional corrosive process of the piece causing the elimination of the structure. The corrosion process is defined as a destructive process that causes a deterioration in the material as a result of a chemical attack caused by the environment.

In the biodesintegration process using the bacteria described in the present invention, it is used to eliminate metal scrap, whose waste is generated because it has reached its useful life, the metal structure has undergone a destructive and functional corrosive process of the piece causing the elimination of the structure. The corrosion process is defined as a destructive process that causes deterioration in the most common ways in which corrosion is generated is through a chemical reaction, which are named below the material as a result of a chemical attack caused by the environment:
- **Oxidation Reaction:** it is generated from a metal having free electrons.
- **Reduction Reaction:** consisting in the consumption of electrons generated in the oxidation reaction.
- **Global Reaction:** the oxidation reaction that is in which electrons are generated and the reduction reaction that is in which they are consumed, must occur at the same time and at the same level for the electrochemical reaction to take place.

Because corrosion is the result of a chemical reaction, there are various conditions that affect the amount of corrosion that an element acquires, among these conditions are: temperature, type of environment, the stresses to which the element is subjected and the erosion. The different forms of corrosion in metal are described below:
1. **Uniform attack corrosion:** characterized because it occurs as a result of an electrochemical or chemical reaction on the entire surface of the material, which has been exposed to a corrosive environment. This attack represents the greatest corrosion problem that metals present.
2. **Galvanic corrosion:** It occurs because two metals in contact with different electrochemical potentials are exposed to a corrosive environment. This is mainly due to the relationship between the anode and cathode area. If the ratio of the anode area is very large in relation to the cathode area, corrosion is rapid. If the ratio of the anode area is small in relation to the cathode area, the corrosive attack occurs slowly.
3. **Pitting Corrosion:** This type of corrosion is found in a specific area, that is, the attack occurs only in certain areas of the material and produces holes or pits.
4. **Crevice Corrosion:** type of localized electrochemical corrosion that occurs in those spaces (cracks) that are formed when joining two materials, in the same way they occur in a place where some type of solution or liquid stagnates.
5. **Intergranular corrosion:** type of localized corrosion that occurs at the grain boundaries of the material. Under normal conditions, if a metal suffers from uniform corrosion, the grain boundaries will only be slightly more reactive than the matrix, but under other conditions, the grain boundaries can be very reactive, resulting in intergranular corrosion that would bring about as a consequence the loss of resistance of the material and even disintegration of the grain boundaries.
6. **Stress corrosion:** It occurs when the material is subjected to a certain stress in a corrosive environment, which causes the material to break or fracture.
7. **Erosion Corrosion:** acceleration of the corrosion process in a material due to a corrosive substance moving on the material.
8. **Corrosion by selective attack:** its main characteristic, only one of the elements that make up the alloy is preferentially attacked.
9. **Corrosion by bacterial attack:** this type of corrosion is not described in the literature, but is nevertheless described in the present patent application. This type of corrosion is due to the exposure of the metal on a solution rich in bacteria that oxidize the metal directly (bacteria feed on the chemical composition of the metal) and indirectly (corrosion by erosion) due to the fact that the bacterial solution is Highly oxidizing and by chemical effect it generates the corrosion of the metal. This type of corrosion is highly accelerated by the direct effect generated by the bacteria present in the oxidizing solution, generating a highly oxidizing solution.

Therefore, any structure that has undergone a process of structural corrosion of the metal is considered industrial waste, which can be treated by the bio-disintegration process described in the present invention.

The present invention provides a method that allows the biodesintegration of industrial metal scrap using a group of microorganisms that comprises a product 1 that contains a consortium of microorganisms adapted to high concentrations of ferrous sulfate (FeSO₄) and ferric sulfate Fe₂(SO₄)₃, in an acidic and inorganic environment that favors the disintegration of industrial metal waste that pollutes the environment, a product 2 that contains an intermediate solution of product 1 and allows the removal of surface oxide from a metal structure and a product 3 that contains a solution generated from the oxidation of product 1 being a highly oxidizing agent with application in the hydrometallurgy process in the extraction of copper.

### Example 1: Obtaining a microbial consortium

The EI Tatio Geothermal Field located in the Andean highlands of northern Chile at 4,200 m.a.s.l., is one of the highest geyser areas in the world. Approximately 80 active geysers have been documented, being the largest geyser field in the southern hemisphere and the third largest in the world, preceded by Yellowstone National Park, United States, and The Valley of Geysers in Kamchatka, Russia (Glennon and Pfaff, 2003). It is a complex geothermal field, which allows the development of microbial communities such as bacteria and archaea, which live under unique and extreme environmental conditions, including a varied temperature range, intense ultraviolet radiation, the presence of metals, metalloids and high concentrations of salts. (Phoenix et al., 2006; Alsina, 2013). The diversity of the species that exist in a given habitat is a consequence of the relationship between organisms and the environment. From an ecological point of view, it is of interest to know why this diversity exists, how they are organized in the microbial community and what value it has for the structure and function of the entire community (Zahawi et al., 2005).

The samples were collected from different points of the EI Tatio Geothermal Field (Figure 2), where some physical and chemical parameters of the samples were determined. The pH (1.0 to 3.5), temperature (Range: 18 ° C to 45 ° C) and color of the samples were measured. At each sampling point, sediment and solution were obtained by storing them in a sterile 50 ml Falcon tube. Subsequently, a direct observation under the microscope was carried out to identify the presence of microorganisms, finally they were stored at 4 ° C, in order to maintain the metabolic activity of the bacteria.

In order to obtain the greatest amount of microorganisms grouped in one sample, all the samples obtained from the EI Tatio Geothermal Field were mixed to generate a single sample, which was cultured in an enriched medium for the development of chemolyttrophic microorganisms, with the addition of of basal salts and the use of ferrous sulfate (FeSO₄) as an energy source in an aerobic environment, with agitation and at temperature (Range; 18°C to 45°C). The microorganisms were cultured in 9K medium, formulated by; 0.04 g · L⁻¹ K₂HPO₄, 0.4 g · L⁻¹ MgSO₄ and 0.1 g · L⁻¹ NH₄SO₄, 33.3 g · L⁻¹ of FeSO₄ · 7 H₂O, with a gradual decrease in pH until adaptation of microorganisms to a range of pH 1.0 - 3.5 (acidified with H₂SO₄ at 98%). The growth curves were performed by cell counting in Neubauer chambers.

The tests were carried out in 250 mL flasks, with a 50 mL working volume and the addition of 10% (v/v) inoculum. As a positive control, the strain of microorganisms acquired in the Campo Geothermal EI Tatio sample was used; negative controls were performed for each test without the addition of microbial inoculum (abiotic controls). The tests were carried out at a temperature that varied between 18ºC-45ºC, with variable agitation where the sample was monitored for 30 days. To evaluate the growth of the consortium in each trial, microscopic visualization and cell counts were performed in a Neubauer chamber every 24 hours. Each trial was performed in triplicate.

From the samples from the Tatio geothermal field, it was possible to obtain a consortium of iron oxidizing microorganisms, they were able to adapt to the final cultivation conditions; 9K medium enriched with ferrous sulfate (FeSO₄), at a range of pH 1.0 to 3.5 at a temperature in a range of 18 °-45 ° C. The oxidation of Fe²⁺ ions to Fe³⁺ was observed by the physical change of the culture medium, which presented color changes to reddish-brown tones (Image 3), representative of the presence of Fe³⁺ ions (Manning, HL (1975). New medium for isolating iron-oxidizing and heterotrophic acidophilic bacteria from acid mine drainage. Applied microbiology, 30 (6), 1010-1016). The oxidation of Fe²⁺ ions was attributed to microbial action, since this phenomenon was not recorded in the corresponding abiotic controls. The results of the microbial kinetics show an exponential phase reaching a population of 10⁸ cells/mL.

### Example 2: Microscopic characterization of cultured microorganisms

Different observations were made under microscopy, in order to obtain a first approximation of the morphology of the microorganisms present and to determine if they are mobile, the procedures used are:
**Slop Drop Method:** This procedure is the simplest way to observe living organisms, it provides information on the morphology, size, color and mobility of the bacteria. However, due to the lack of contrast with the environment, it is a practice that is used only for the observation of movement.

Take a drop (10 µl) in a liquid culture using a sterile Pasteur pipette, it was placed in the center of a coverslip, then the excavated slide was placed on the coverslip and a drop of immersion oil was placed, in order to observe with objective (100x).

This technique allows observing the fast and rectilinear movement or turning and turning in those bacteria that have flagella. Immobile bacteria present a vibratory movement called Brownian movement, due to the collision of molecules in a liquid solution

**Gram Stain:** Differential staining that was proposed by the Danish physician Christian Gram (1884) (Tortora et al., 2007). Differential staining most commonly used routinely and practically the first test that samples of any origin are subjected to prior to study. It provides essential information, in addition to the shape, size and cell grouping, such as the type and composition of the wall that the bacteria present. The Gram stain divides the bacteria with a wall of the Bacteria Domain into two large groups: bacteria with a positive Gram wall type and bacteria with a negative Gram wall type. The methodology consists of placing a drop of the bacterial culture on the slide with a micro pipette. Subsequently, make a smear, forming a homogeneous film on the slide with the seeding loop. Let dry. Fix the preparation by passing the slide through the flame of the burner, then cover with a few drops of crystal violet for 1 minute. Then the excess dye must be washed with sterilized demineralized water. Subsequently, the Lugol mordant, iodine-potassium iodide solution, was added for 1 minute, then the excess of the mordant was washed with sterile distilled water. The preparation was then washed with alcohol at an angle to the preparation, for 30 seconds (discoloration time is key for a correct result) and then it was necessary to wash immediately with plenty of water. Finally the sample was covered with the contrast dye, safranin for 1 minute, then the excess dye was washed with water and allowed to air dry. In order to observe the sample, add a drop of immersion oil.

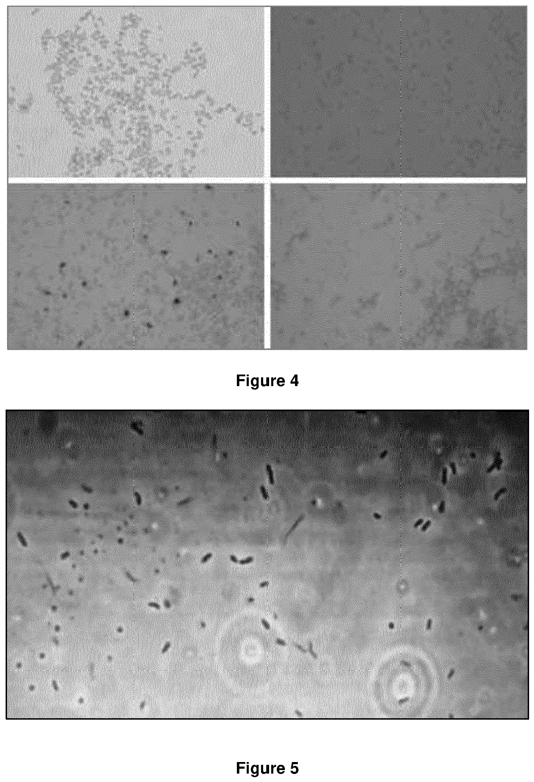

Observe with an immersion objective (100x). The different structure and organization of the cell wall in these two types of organisms makes both groups respond differently, thus while Gram positive bacteria maintain and conserve the crystal violet-iodine complex, Gram negative bacteria quickly discolor with application. of alcohol, admitting the contrast dye that provides a color between pink and red that contrasts with the intense violet color (Figure 4).

Different explanations have been proposed to justify the response of microorganisms to this staining, it seems that the difference is due both to the physical structure and the composition of the cell wall, in Gram positive bacteria the thick layer of peptidoglycan with abundant cross-links seems to contribute to the retention of the fundamental dye, crystal violet, while in Gram negative bacteria the thin layer of peptidoglycan together with the abundance of lipids in the outer membrane of the cell wall increase porosity and therefore contribute to the loss of the fundamental dye after of bleaching with alcohol (Figure 4).

In the microscopic characterization, a general view of the study sample was obtained by confocal microscopy, allowing to elucidate the mobility capacity (Slope drop method) morphology (Gram stain) Figure 4. The observation by electron microscopy (STEM) revealed the presence of bacillus-like morphologies, found in isolation or in diplobacilli and with variable sizes. By Gram staining it was determined that the bacterial consortium is composed of gram-negative microorganisms (Table 4), which agrees with reports where gram-negative microorganisms are described as the most frequent in geothermal fields (Garrity, GM, Bell, JA and Lilburn, TG (2004). Taxonomic outline of the prokaryotes Bergey's Manual of Systematic Bacteriology. Second edition. Ed. Sringer. New York. USA).

### Example 3: Extraction of total genomic DNA

The genomic DNA was obtained from the bacterial inoculum generated from the environmental samples obtained in the EI Tatio Geothermal Field, whose samples were mixed and cultivated in the 9K culture medium enriched with ferrous sulfate (FeSO₄) adjusting the pH 1.0 to 3.5, with aeration and constant agitation. Once all the ferric sulfate (FeSO₄) had been oxidized to ferric sulfate Fe₂(SO₄)₃ and the number of bacteria in the sample was 10⁸ cells/ml, the samples were taken to extract the genomic DNA.

The purification of the samples was carried out with PowerSoil^{®} DNA Isolation Kit (MO BIO Laboratories, Carlsbad, CA, USA) according to the manufacturer's instructions. The purified DNA was quantified in an Epoch spectrophotometer (Bio-Tek Instruments Inc, Winooski, VT, USA), and its purity was verified by the absorbance ratio at 260 nm and at 280 nm (A_{260/280}). For the identification of bacterial species, the 16S ribosomal DNA (rDNA) was amplified using the universal oligonucleotides 1492R/27F and the GoTaq^{®} Green Master Mix PCR kit (Promega, Madison, WI, USA). The amplification program was as follows: an initial denaturation at 94 ° C for 5 minutes, followed by 35 cycles at 94 ° C for 45 s, 57 ° C for 45 s, and 72 ° C for 1 minute 30 s; with a final extension at 72ºC for 5 minutes. To verify the extraction of genomic DNA and its status, 7 µl were loaded on a 1% agarose gel, an electrophoretic run was performed and it was finally visualized in a UV light transilluminator (Vilber Lourmat ECX-F20.M). The DNA obtained was quantified using Nanodrop (Thermo).

### Example 4: 16S rRNA gene polymerase chain reaction (PCR) and agarose gel electrophoresis.

The extracted DNA was used as a template to amplify the bacterial 16S ribosomal gene (~ 1,500 bp) from the sample of the bacterial inoculum to be identified. The primers 27F and 1542R specific for the Bacteria domain were used (Stackebrandt et al., 1993) (Table 2). The PCR reaction was carried out in a total volume of 25 µL, containing 14.55 µL of MilliQ water, 5 µL Buffer GoTaq (5 x), 1.7 µL MgCl₂ (1.7 mM), 0.5 µL dNTPs ( 2 mM), 1 µL 27F primer (0.8 mM), 1 µL 1542R primer (0.8 mM), 0.25 µL DNA polymerase GoTaq (1.25 U), and 1 µL annealed DNA. The PCR protocol started with initial denaturation at 94 ° C for 5 minutes, followed by 30 cycles of denaturation at 94 ° C for 45 (s), alignment at 54 ° C for 45 (s). and extension at 72 ° C for 1.30 minutes, finally carrying out an extension at 72 ° C for 5 minutes. (Stackebrandt et al., 1993). The visualization of the amplicons was carried out by electrophoresis in a 1.0% agarose gel, stained with ethidium bromide, at 120 V for 30 minutes.

**Table 3. Primers used in molecular analysis of the sample**

| Oligonucleotide name | Domain | Position | Sequence (5'-3') | Reference |
|---|---|---|---|---|
| 27F | Bacteria | | SEQ ID NO: 1. A GA GTT TGA TCC TGG CTC A G | Stackebrandt & Liesack (1993) |
| 1542R | Bacteria | | SEQ ID NO: 2. A GA A AGGAGGTGA TC CAG CC | Stackebrandt & Liesack (1993) |
| P3 | Bacteria | 111-130 | SEQ ID NO: 3. GGA A TCTTC CAC A A TGGGCG | Muyzar y cols. (1993) |
| P2 | bacteria | 361-380 | SEQ ID NO: 4. TTC CCC A CGCGT TAC TCA CC | Muyzar y cols. (1993) |

### Example 5: Evaluation of the microbial community under study by denaturing gradient gel electrophoresis (DGGE).

The DGGE analysis was performed according to Casamayor et al., (2003) with PCR products of the 16S rRNA gene of bacteria generated with the P2 / P3 oligonucleotides (Muyzer et al., 1993) (Table 2). The PCR products were placed on 7.5% polyacrylamide gels containing a linear denaturing gradient of 30-60% for bacteria where 100% denaturing was defined as 7M urea and 40% formamide (Sigma). The DGGE run was performed in the BioRad D Gene system (BioRad) at 60 ° C, 200 V, for 6 hours. The gels were developed with SYBR Gold (Invitrogen). For development of the DGGE gels with SYBRGold, the DGGE plates were carefully removed and the polyacrylamide gel was placed on the tray for staining using SYBRGold at a final concentration of 2.5 x. The gel was covered with SYBRGold and kept for 1 hour in the dark. Later the bands were observed under UV light in the transilluminator.

The number of taxonomic units in each sample was defined according to the number of bands obtained in the DGGE (Muyzer, 1993). Cut bands were reamplified and sent to sequence.

### Example 6: Bioinformatic and Phylogenetic analysis.

With the information of the 16S rRNA sequence, the microbial groups that were present in the analyzed samples were established; However, before establishing the microbial groups of the analyzed samples, different bioinformatic platforms were used. First, these sequences were cleaned with the ChromasPro program (Table 3). The edited sequences were then compared with the sequences available in the database, using the NCBI Blastn program (Altschul, SF, Gish, W., Miller, W., Myers, EW (1990). Basic local alignment search tool. Journal of Molecular Biology 215 (3), 403-410). Once the Bacteria species had been identified, the closest corresponding genome was searched in the NCBI database (access to genomes). Subsequently, the sequences obtained in NCBI were compared, using the RDP database (Table 3)

**Table 3. List of applications and bioinformatics resources for the analysis of DNA sequences used.**

| Web application | Analysis and tools | Internet way |
|---|---|---|
| NCBI | Gene search | www.ncbi.nlm.nih.gov |
| BLASTN | Similarity | www.ncbi.nlm.nih.qov/blastn |
| RDP | Similarity | http://rdp.cme.msu.edu/classifier.jsp |
| CHROMAS PRO | Editing sequences | http:/en.bio-soft.net/dna/chromas.html |

### Example 7: Adaptation of the bacterial consortium to different concentrations of ferrous sulfate (FeSO₄) and Ferric sulfate Fe₂(SO₄)₃

Resistant bacteria are those that can thrive under adverse environmental conditions, in which most organisms could not survive. These types of adaptations allow its selective isolation in the presence of the compound of interest. Thanks to this, bacterial cultures can be obtained on certain selective media.

From the environmental samples obtained from the EI Tatio Geothermal field, they were grown in 250 ml flasks with 9K culture medium, formulated by; 0.04 g · L⁻¹ K₂HPO₄, 0.4 g · L⁻¹ MgSO₄ and 0.1 g · L⁻¹ NH₄SO₄, 33.3 gL⁻¹ of FeSO₄ · 7H₂O with the gradual decrease of the pH until the adaptation of the microorganisms at a range of pH 1.0 - 3.5 (acidified with H₂SO₄ at 98%) at a variable agitation between 50 to 200 rpm and the temperature varied between 18 ° C to 45 ° C for 30 days initially. Once the solution reached a Redox potential over 700mV vs Ag/AgCl and the number of bacteria exceeded 10⁸ cells/ml. The solution was already ready to perform a re-culture of the bacterial inoculum, a procedure that allows the microorganisms to partially adapt to the new environment. For the re-cultivation of the microorganisms in fresh medium, it was necessary to recover all the cells generated in the first culture, for this a vacuum filter was used, the filtration equipment contained a Millipore nitrocellulose membrane of 0.2 µm of diameter. Once the total solution was filtered, the membrane was washed without disassembling the filtration equipment, with fresh culture medium. Finally, the membrane used was placed in the fresh culture medium and vigorously shaken to transfer the largest number of cells from the membrane to the fresh culture medium and start the new initial adaptation cycle, this procedure was repeated 15 times with the purpose of having a partial adaptation optimizing the time of the bacterial growth cycle, reaching its stationary phase on the third day. The results obtained at the end of the 15 re-culture cycles, was a bacterial inoculum with the ability to oxidize all ferrous sulfate (FeSO₄) to Ferric sulfate Fe₂(SO₄)₃, ending with a Redox potential over 700mV vs Ag/AgCl and the number of bacteria exceeded 10⁸ cells / ml, in just 3 days, under previously established conditions.

Subsequently, the bacterial growth was scaled up in the same culture conditions indicated above, starting the scaling in 1-liter, 2-liter flasks, in 20-liter bioreactors and in 1000-liter capacity ponds, as a result a bacterial inoculum was obtained with the ability to oxidize all ferrous sulfate (FeSO₄) to Ferric sulfate Fe₂(SO₄)₃, ending with a Redox potential of over 700mV vs Ag/AgCl and the number of bacteria exceeded 10⁸ cells/ml, in just 3 days. Once the process of generating the bacterial inoculum of interest had been scaled up, the adaptation tests were continued partially to high concentrations of Ferrous Sulfate (FeSO₄) and consequently to the adaptation of Ferric sulfate Fe₂(SO₄)₃, generated by the bacterial action in the culture medium, which transformed all the Fe⁺² in the culture medium into Fe⁺³.

Next, the procedure used for the gradual adaptation of bacteria to different concentrations of ferrous sulfate (FeSO₄) is described, in order to increase the resistance of bacteria to Fe⁺² and Fe⁺³, a condition that will allow them to bacteria develop, grow and survive at high concentrations Iron, in order to optimize the oxidation process of metal scrap as much as possible.

The process of adaptation of bacteria to high concentrations of ferrous sulfate (FeSO₄) began with a concentration of 30% (initial conditions necessary for the normal growth of leachate bacteria), later an increase in the concentration of ferrous sulfate was carried out (FeSO₄) in 10% w/v in the culture medium used for each experimentation cycle until reaching the maximum tolerance (Table 4).

**Table 4. Cycles of bacterial adaptation to an increase in ferrous sulfate (FeSO₄) in the culture medium**

| Cycles of Adaptation | FeSO₄ % concentration |
|---|---|
| 1 | 30 |
| 2 | 40 |
| 3 | 50 |
| 4 | 60 |
| 5 | 70 |
| 6 | 80 |
| 7 | 90 |
| **8** | **100** |
| 9 | 110 |
| 10 | 120 |
| 11 | 130 |
| 12 | 140 |
| 13 | 150 |
| 14 | 160 |
| 15 | 170 |
| 16 | 180 |

| | |
|---|---|
| *cycle of adaptation intermediate | |

**Bacterial adaptation cycles:** each adaptation cycle varies with the time required to oxidize the entire concentration of ferrous sulfate in the culture medium, therefore, it is expected that the greater the amount of ferrous sulfate, the longer its adaptation time.

### Stage 1: Primary inoculation

250 ml flasks are prepared, 100 ml of total solution is prepared, this solution contains 95% of the culture medium and 5% of the bacterial inoculum generated in the previous step. The culture medium begins with the base ferrous sulfate concentration (30% w/v) of the 100 ml.

### Stage 2: Sample monitoring

Bacterial growth is evaluated daily by quantifying the solution by counting in the 0.01 mm Neubauer chamber (Neubauer- improved, Marienfeld), the concentration of ferrous sulfate (FeSO₄) is quantified using a titration method, potential measurement is performed reduction oxide (Eh) using a potentiometer and pH measurements were performed using a pH meter.

### Etapa 3: Fase estacionaria del crecimiento bacteriano

In order to determine when the adaptation process was ready to continue with the next adaptation cycle, it was necessary that the final solution had the appropriate requirements, which are described below: Number of microorganisms in the solution had to be greater than 10⁸ cells/ml, Potential Oxide reduction greater than 700 mV and the concentration of ferrous sulfate (FeSO₄) should be less than 1 g/ml.

It is worth mentioning that as the concentration of ferrous sulfate (FeSO₄) in solution increased, the oxidation process took longer than the established time, due to this, the adaptation procedure (Stage 1, Stage 2 and Stage 3), was repeated as many times as necessary to optimize the total oxidation time in three days. Upon reaching the adaptation cycle No. 8, equivalent to the adaptation of the bacterial inoculum of 100% w/v of ferrous sulfate (FeSO₄) in the culture medium used, the intermediate adaptation process consisted in disposing the culture medium with the addition of the Bacterial inoculum from the previous adaptation cycle (bacteria adapted to 90% w/v of the culture medium) plus the addition of ferrous sulfate (FeSO₄) to 100% w/v of the culture medium. In this cycle, the adaptation process went through multiple re-cultures, in order to have a reinforced and very well adapted bacterial inoculum, with the ability to tolerate higher concentrations of ferrous sulfate (FeSO₄), additionally microscopic visualization was performed, to determine the activity (mobility) and morphology of the bacteria (Figure 5).

### Example 8: Biodesintegration of metal scrap, by the use of the bacterial consortium adapted to high concentrations of ferrous sulfate (FeSO₄) and Ferric sulfate Fe₂(SO₄)₃

In order to evaluate and confirm the bio-disintegration effect of metal scrap using the bacteria characterized in the present invention, the way in which the piece loses weight due to corrosion will be the experimental technique, this type of test is the most used at present due to its great efficiency and the simplicity to carry it out. This technique consists of exposing the piece in a test environment for a certain period of time and then evaluating the amount of material that was lost, the time the piece is exposed, the weight loss will be the parameter considered to carry out this test and obtain the amount of metal corrosion.

The experimental development consisted of carrying out three tests in triplicate, where different corrosion scenarios of a metallic piece were evaluated to determine the effect generated by bacteria adapted to high concentrations of ferrous sulfate (FeSO₄). For this evaluation, a metal piece was cut into 9 equal parts with a diamond disc cutter, in order to obtain homogeneous pieces, subsequently the weight of each piece was determined. **Sample 1 or negative control 1:** a 1000 ml beaker was used as a container for the metal piece, which was kept completely submerged in the control solution (acidified distilled water at a pH ranging from 1.6 to 3.5) , was kept under constant stirring and at a temperature that varied between 18°C to 45ºC for 7 days. To carry out the test, a metallic piece with an initial weight of 100.05 (g) was selected.

**Sample 1** 'A 1000 ml beaker was used as a container for the metal piece, which was kept completely submerged in the control solution (distilled water acidified to a pH ranging between 1.6 to 3.5) and kept under stirring. constant and at a temperature that varied between 18°C to 45ºC for 7 days. To carry out the test, a metallic piece with an initial weight of 100.12 (g) was selected.

**Sample 1**": a 1000 ml beaker was used as a container for the metal piece, which was kept totally submerged in the control solution (distilled water acidified to a pH that varies between 1.6 to 3.5). kept under constant stirring and at a temperature that varied between 18°C to 45ºC for 7 days. To carry out the test, a metallic piece with an initial weight of 100.31 (g) was selected.

**Sample 2 or negative control 2:** a 1000 ml beaker was used as a container for the metal piece, which was kept totally submerged in culture medium plus the inoculum of bacteria adapted to high concentrations of FeSO₄, which contained the following salts (NH₄)₂SO₄, MgSO₄ and K₂HPO₄, adjusted to a pH ranging from 1.6 to 3.5, was kept under constant stirring and at a temperature ranging from 18 ° C to 45 ° C for 7 days. To carry out the test, a metallic piece with an initial weight of 100.00 (g) was selected. **NOTE:** Ferrous sulfate (FeSO₄) was not added in this test so that the bacteria would feed directly on the iron from the metal.

**Sample 2** 'A 1000 ml beaker was used as a container for the metal piece, which was kept totally submerged in culture medium plus the inoculum of bacteria adapted to high concentrations of FeSO₄, which contained the following salts (NH₄)₂SO₄, MgSO₄ and K₂HPO₄, adjusted to a pH that varies between 1.6 and 3.5, was kept under constant stirring and at a temperature that varied between 18°C to 45ºC for 7 days. To carry out the test, a metallic piece with an initial weight of 101.02 (g) was selected. **NOTE:** Ferrous sulfate (FeSO₄) was not added in this test so that the bacteria will feed directly on the iron from the metal.

**Sample 2":** a 1000 ml beaker was used as a container for the metal piece, which was kept totally submerged in culture medium plus the inoculum of bacteria adapted to high concentrations of FeSO₄, which contained the following salts (NH₄)₂SO₄, MgSO₄ and K₂HPO₄, adjusted to a pH that varies between 1.6 and 3.5, was kept under constant stirring and at a temperature that varied between 18°C to 45ºC for 7 days. To carry out the test, a metallic piece with an initial weight of 100.58 (g) was selected. **NOTE:** In this test, ferrous sulfate (FeSO₄) was not added so that the bacteria would feed directly on the iron in the metal.

**Sample 3 or negative control 3:** a 1000 ml beaker was used as a container for the metal piece, which was kept completely submerged in an oxidizing solution in the presence of bacteria adapted to high concentrations of Fe₂(SO₄)₃, the solution used had already oxidized all the FeSO₄ to Fe₂(SO₄)₃ by bacterial action. The oxidizing solution contained the following salts FeSO₄, (NH₄)₂SO₄, MgSO₄ and K₂HPO₄, adjusted to a pH that varies between 1.6 to 3.5, the solution was kept under constant stirring and at a temperature that varied between 18°C to 45ºC for 7 days. To carry out the test, a metallic piece with an initial weight of 101.31 (g) was selected.

**Sample 3':** a 1000 ml beaker was used as a container for the metal piece, which was kept totally submerged in an oxidizing solution in the presence of bacteria adapted to high concentrations of Fe₂(SO₄)₃, the solution used had already oxidized all FeSO₄ to Fe₂(SO₄)₃ by bacterial action. The oxidizing solution contained the following salts FeSO₄, (NH₄)₂SO₄, MgSO₄ and K₂HPO₄, adjusted to a pH that varies between 1.6 to 3.5, the solution was kept under constant stirring and at a temperature that varied between 18°C to 45ºC for 7 days. To carry out the test, a metallic piece with an initial weight of 100.91 (g) was selected.

**Sample 3':** a 1000 ml beaker was used as a container for the metal piece, which was kept totally submerged in an oxidizing solution in the presence of bacteria adapted to high concentrations of Fe₂(SO₄)₃, the solution used had already oxidized all FeSO₄ to Fe₂(SO₄)₃ by bacterial action. The oxidizing solution contained the following salts FeSO₄, (NH₄)₂SO₄, MgSO₄ and K₂HPO₄, adjusted to a pH that varies between 1.6 to 3.5, the solution was kept under constant stirring and at a temperature that varied between 18°C to 45ºC for 7 days. For Sample 3": a 1000 ml beaker was used as a container for the metal piece, which was kept totally submerged in an oxidizing solution in the presence of bacteria adapted to high concentrations of Fe₂(SO₄)₃, the solution used had already oxidized all the FeSO₄ to Fe₂(SO₄)₃ by bacterial action. The oxidizing solution contained the following salts FeSO₄, (NH₄)₂SO₄, MgSO₄ and K₂HPO₄, adjusted to a pH that varies between 1.6 to 3.5, the solution was kept under constant stirring and at a temperature that varied between 18º to 45ºC for 7 days. To carry out the test, a metallic piece with an initial weight of 101.50 (g) was selected; a metallic piece with an initial weight of 100.91 (g) was selected.

Next, the daily weights of the metals treated with the different experimental solutions are described:

**Table 5. The weight of the metal in grams (gr) was determined daily for 7 days. M1: negative control, M2: culture medium 9K + adapted bacterial inoculum FeSO₄, M3: culture medium 9K + adapted bacterial inoculum Fe₂(SO₄)₃.**

| | Day 1 | Day 2 | Day 3 | Day 4 | Day 5 | Day 6 | Day 7 |
|---|---|---|---|---|---|---|---|
| M1 | 100,05 | 99,12 | 98,99 | 98,75 | 98,46 | 97,63 | 97,11 |
| M1' | 100,12 | 99,89 | 98,82 | 98,75 | 98,48 | 97,75 | 97,24 |
| M1" | 100,31 | 100,16 | 99,58 | 99,12 | 98,45 | 98,10 | 97,61 |
| M2 | 100,00 | 94,12 | 88,47 | 84,75 | 79,66 | 75,59 | 71,43 |
| M2' | 101,02 | 94,57 | 90,78 | 86,42 | 82,87 | 79,55 | 75,81 |
| M2" | 100,58 | 94,92 | 81,63 | 78,12 | 75,39 | 72,15 | 70,18 |
| M3 | 101,31 | 86,11 | 68,88 | 58,75 | 49,03 | 41,52 | 35,21 |
| M3' | 100,91 | 86,78 | 73,16 | 62,18 | 52,85 | 44,44 | 37,29 |
| M3" | 101,50 | 86,20 | 73,01 | 62,35 | 52,99 | 43,49 | 36,96 |

**M1; M1 and M1**": They were negative controls, metal corrosion was observed because the solution had an acid pH, a parameter that of metal corrosion, however, the effect was low because at the end of the test the Average weight of the three samples reached a decrease of 2.84% of the weight of the metal at the end of day 7 of experimentation.

**M2, M2' and M2**": They were the tests that were carried out to determine the effect of corrosion generated by the bacteria in the culture medium without ferrous sulfate (FeSO₄) and adjusted to an acid pH, with the purpose that the bacteria obtained their source energetically directly from the iron of the metal. The results obtained determine a metal corrosion greater than the negative control without bacteria, where the final average weight after 7 days of experimentation decreased by 27.91%.

It is concluded that this experimental solution presented a partial corrosion due to the fact that the bacteria took longer to disintegrate the metal due to the fact that their energy source had to be acquired from the same metallic piece. However, it is possible to mention that the solution was able to eliminate the entire surface that had an oxidation state, which would allow to have a biological solution to clean metal surfaces that are in a partial oxidation state.

**M3; M3' and M3":** They were the most important tests of the experimentation, in order to determine the biodesintegration effect of a metal part generated by the bacteria in an oxidizing medium characterized in the present invention. The results obtained were very promising because the weight of the metal in experimentation was an average loss of 63.95% at the end of day 7 of experimentation.

**M3; M3' and M3":** They were the most important tests of the experimentation, in order to determine the biodesintegration effect of a metal part generated by the bacteria in an oxidizing medium characterized in the present invention. The results obtained were very promising due to the fact that the weight of the metal in experiment. Due to the fact that the bacteria present in the experimental solution transformed the vast majority of its energy source (FeSO₄) to Fe₂(SO₄)₃ obtaining a highly oxidizing solution, which facilitated the metal corrosion process, Figure 6 was an average loss of 63.95% at the end of day 7 of experimentation.

Due to the results obtained in this test at the laboratory level, which determines the biodesintegration of metal scrap using a bacterial consortium adapted to high concentrations of ferrous sulfate (FeSO₄) and ferric sulfate Fe₂(SO₄)₃, where it is obtained as resulting in the removal of metal weight over 60% in just 7 days. A scalable method is proposed to be able to process large quantities of metal scrap, in order to eliminate this type of industrial waste and offer a new commercial product.

### Example 9: Bioleaching experiments using the bacterial consortium of the present invention, which has the characteristic of biodesintegration of metal scrap.

**Inoculum used:** the bioleaching solution used in the following experimental test was the product obtained after Biodisintegration of metal scrap by the action of bacteria adapted to high concentrations of ferrous sulfate (FeSO₄) and ferric sulfate (Fe₂(SO₄)₃), characterized in the present invention. The characteristics of this solution are: Number of leachate microorganisms greater than 10⁸ cells/ml, Oxide Potential reduction greater than 700 mV, Acid pH, which varied between 1.0 to 3.5 and a concentration of ferric sulfate (Fe₂(SO₄)₃) would have to be the vast majority. These qualities generated from the biotransformation of metal scrap give the bacterial inoculum a better quality as an oxidizing agent, which significantly improves the extraction of copper from sulphide minerals.

**Mineral to be used:** Bioleaching tests using the final solution after the biotransformation of the metal scrap, were carried out in duplicate in 1.5 meter columns. Each 18 cm diameter acrylic column containing 50 kilos of primary sulfide mineral (47% Chalcopyrite, 22% Bornite, 16% Pyrite, 12% Chalcosine, 2% Covelina and 1% High-grade Tenantite (0.96% Cu) The mineral to be used was homogenized by shoveling technique. Subsequently, by means of the cone and quartering methodology, 6 parts were divided, using 50 kilos of the miner per column (6 columns), for each sample 400 grams was taken to determine the moisture of the mineral and determine the composition and type of copper (Table 6).

**Table 6. Global chemical analysis of the mineral used**

| Matraz | CuT (%) | AsCu (%) | CNsCu (%) | Cu residual (%) | FeT (%) | AsFe (%) |
|---|---|---|---|---|---|---|
| 1 | 0,95 | 0,36 | 0,53 | 0,06 | 4,20 | 0,16 |
| 2 | 0,96 | 0,38 | 0,51 | 0,07 | 4,21 | 0,23 |
| 3 | 0,96 | 0,36 | 0,55 | 0,05 | 4,05 | 0,16 |
| 4 | 0,95 | 0,34 | 0,54 | 0,07 | 4,23 | 0,16 |
| 5 | 0,97 | 0,37 | 0,52 | 0,08 | 4,19 | 0,12 |
| 6 | 0,96 | 0,34 | 0,54 | 0,08 | 4,15 | 0,17 |

**Agglomeration and Curing:** each load of mineral was carried out by adding process water, sulfuric acid and bacterial inoculum, according to Table 7. The resting time of the mineral was 7 days, since the columns were loaded at the beginning irrigation.

**Table 7. Agglomerate condition and curing of each column. Column 1 and 2 represent the control tests, Columns 3 and 4 are experimental tests, where the mineral was agglomerated in a traditional way and a bacterial strain used traditionally in the Bioleaching process (Inoculum 1) and Column 5 and 6 the The mineral was agglomerated in the traditional way and a solution obtained after the bio-disintegration process of the metal scrap was added, where bacteria adapted to high concentrations of ferrous sulfate (FeSO₄) and ferric sulfate (Fe₂(SO₄)₃) are used (Inoculum 2).**

| Mineral Wetting | |
|---|---|
| Column 1 | Process solution + H₂SO₄ |
| Column 2 | Process solution + H₂SO₄ |
| Column 3 | Process solution + inoculum 1 + H₂SO₄ |
| Column 4 | Process solution + inoculum 1 + H₂SO₄ |
| Column 5 | Process solution + inoculum 2 + H₂SO₄ |
| Column 6 | Process solution + inoculum 2 + H₂SO₄ |

**Leaching Stage:** In the first instance, irrigation was irrigated with an intermediate copper solution at an irrigation rate of 8 L/m²h up to a leaching rate of 1.6 m³/tm and, later, with a low copper solution, at an irrigation rate of 5 L/m²h by means of an open circuit circulation system.

**Monitoring:** The daily monitoring of the tests included the physical, chemical and biological analyzes of the feed solutions and percolates during the operation of the columns, represented by: total copper (CuT), Sulfuric acid (H₂SO₄), Ferrous Ion (Fe II), Ferric Ion (Fe III), Total iron (Fe T), potential of the solution (Eh), pH and Number of cells/ml.

**Column discharge and gravel collection:** Once the column irrigation period is over, irrigate with 3 liters of water, simulating a washing stage to dissolve the remaining copper. The cuttings were unloaded and representative samples were taken. The samples were left to dry at 90 ° C for 12 hours and were subsequently crushed and pulverized to send the samples to determine their final chemical composition. The final copper recovery results are displayed in Figure 7.

Columns 1 and 2 were the negative controls where the mineral is agglomerated in the traditional way, adding a process solution acidified with sulfuric acid (H₂SO₄), where the results were those expected in sulfur mineral, obtaining over 20% in copper recovery at the end of the leaching process.

Columns 3 and 4 represent experimental tests where an inoculum (inoculum 1) was added in the mineral agglomeration process, which contained a mixture of bacteria traditionally used in Bioleaching tests, where it allowed to increase the recovery percentage of copper up to 50% at the end of the leaching process.

Based on the final results obtained in column 5 and 6, the total copper recoveries that exceeds 80% at the end of the leaching process stand out, where the mineral used was agglomerated with the solution obtained after the biodesintegration processes of metal scrap, whose solution has bacteria adapted to high concentrations of ferrous sulfate (FeSO₄) and ferric sulfate (Fe₂(SO₄)₃), in addition whose solution has a high concentration of ferric sulfate (Fe₂(SO₄)₃) (Inoculum 2), which favors the oxidation processes of the mineral.

## Claims

1. Method allowing the biodesintegration of industrial metal scrap using a group of extremophilic microorganisms wherein said extremophilic microorganisms are selected from a consortium of bacteria adapted to high concentrations of ferrous sulfate (FeSO₄) and ferric sulfate Fe₂(SO₄)₃, number Access of the Microbial Genetic Resources Bank, Chile, RGM 2972 of October 16, 2019 of the Chilean Collection of Genetic and Microbial Resources.

2. The method of claim 1 wherein said industrial metal scrap is previously subjected to a cutting procedure if necessary, introduced into a swimming pool or pond for the bio-disintegration process.

3. The method of claim 1 wherein said pool or pond allowing bio-disintegration with aeration, heat or temperature and agitation.

4. The method of claim 3 wherein said consortium of extremophilic bacteria were grown in a 9K culture medium, formulated by; 0.04 g · L⁻¹ K₂HPO₄, 0.4 g · L⁻¹ MgSO₄ and 0.1 g · L⁻¹ NH₄SO₄, and enriched with increasing concentrations of Ferrous Sulfate (FeSO₄), exceeding 180 gpl, with a pH adjusted to a variation between 1.0 to 3.5, with variable shaking between 50 to 200 rpm and at a variable temperature between 18 ° C to 45 ° C, reaching a higher cell growth of 1008 cells/ml.

5. The method of claim 1 wherein said bacterial consortium comprises species of the genus Leptoospirillum sp and Leptoospirillum ferriphilum.

6. Use of a solution comprising a bacterial consortium, access number of the Microbial Bank of Genetic and Microbial Resources RGM 2972 of October 16, 2019 of the Chilean Collection of Genetic and Microbial Resources for eliminating surface corrosion of a metal.

7. The use of claim 6 wherein said bacterial consortium comprising species of *Leptoospirillum sp* and *Leptoospirillum ferriphilum* genus.

8. The use of claim 6 comprising said solution a bacterial consortium at a concentration of 10⁰⁸ cells/ml.

9. The use of claim 7 wherein in said solution said bacterial consortium is found in a culture media 9K (0.04 g· L⁻¹ K₂HPO₄, 0.4 g· L⁻¹ MgSO₄ and 0.1 g· L⁻¹ NH₄SO₄), in absence of ferrous sulfate (FeSO₄), pH adjusted between 1.0 to 3.5, with a variable stirring between 50 to 200 rpm and a variable temperature between 18°C to 45°C.

10. The use of claim 6 to eliminate surface corrosion in a surface or domestic metal device/apparatus and said solution is applied as an aerosol solution on a metal surface o said metal device/apparatus is treated with a solution by bath or immersion or initial irrigation.

11. Use of a biolixiviant solution comprising a bacterial consortium, access number RGM 2972 of 16 October 2019 of the Chilean Collection of Genetic and Microbial Resources in the treatment of metallurgic effluents, as colorant, coagulant to industrial residues, water treatment and metal bioleaching including copper.

12. The use of claim 11 wherein such bacterial consortium comprising species of *Leptoospirillum sp* and *Leptoospirillum ferriphilum* genus.

13. The use of claim 11 wherein said bioleaching comprising bioleaching of oxidized minerals, sulfurates including secondary sulphure, primary sulphure and mixed sulphures or both.

14. The use of claim 11 wherein it can be used in a mineral agglomerate process in *in situ* irrigation, troughs, tanks, reactors, piles or dumps or a combination thereof.
